# EUROPÄISCHE PATENTSCHRIFT

(11) **EP 1 515 769 B1**
(45) Veröffentlichungstag und Bekanntmachung des Hinweises auf die Patenterteilung: **12.03.2014**
(21) Anmeldenummer: 03759964.4
(22) Anmeldetag: 16.06.2003
(51) Int. Cl.: A61M 16/06

(54) **MASKENKISSEN FÜR EINE ATEMMASKE, ATEMMASKE SOWIE VERFAHREN ZUR HERSTELLUNG DERSELBEN**
RESPIRATORY MASK CUSHIONING, RESPIRATORY MASK AND METHOD FOR THEIR PRODUCTION
COUSSIN DE MASQUE RESPIRATOIRE, MASQUE RESPIRATOIRE, PROCEDE POUR LEUR PRODUCTION

(30) Priorität: 14.06.2002 DE 10226587; 26.06.2002 DE 10228554
(43) Veröffentlichungstag der Anmeldung: 23.03.2005
(60) Teilanmeldung: 10184958.6
(73) Patentinhaber: ResMed R&D Germany GmbH, 82152 Martinsried (DE)
(72) Erfinder: LANG, Bernd, 82166 Gräfelfing (DE); BIENER, Achim, 85445 Aufkirchen (DE); BECHTEL, Martin, 21423 Winsen/Luhe (DE)
(74) Vertreter: Heunemann, Dieter
(86) Internationale Anmeldenummer: PCT/EP2003/006334
(87) Internationale Veröffentlichungsnummer: WO 2003/105921

(56) Entgegenhaltungen:
- EP-A- 1 099 452
- WO-A-01/95965

## Beschreibung

### Gebiet der Erfindung

Die Erfindung betrifft ein Maskenkissen für eine Atemmaske, eine Atemmaske an sich sowie ein Verfahren zur Herstellung derselben. Die Erfindung betrifft insbesondere Maskenkissen bzw. eine mit einem derartigen Maskenkissen ausgestattete Atemmaske, durch welche in abdichtender Weise ein Atemmaskeninnenraum im Zusammenspiel mit der Gesichtsfläche eines Maskenanwenders derart gegenüber der Umgebung abdichtbar ist, dass in diesem Atemmaskeninnenraum zumindest phasenweise ein gegenüber dem Umgebungsdruck erhöhter Druck herrschen kann. Derartige Atemmasken finden insbesondere im Zusammenhang mit der medizinischen oder therapeutischen Verabreichung von atembaren Gasen sowie auch im technischen Bereich, zum Beispiel im Bereich der Atemschutztechnik, Anwendung. Die Erfindung befasst sich weiterhin auch mit einer Dichtungsstruktur und einem Verfahren zur Herstellung derselben im allgemeinen.

Üblicherweise wird bei diesen Atemmasken die Abdichtung zur Gesichtsfläche des Atemmaskenanwenders durch eine, um eine Maskenöffnung einwärtsgerichtet umlaufende und aus einem elastisch verformbaren Material gefertigte Dichtlippenstruktur erreicht.

Die mit derartigen Dichtlippen erreichte Dichtwirkung nimmt allgemein mit dem Anpressdruck der Dichtlippe gegen die Gesichtsfläche zu. Im Falle hoher Anpressdrücke kann insbesondere die Langzeitanwendung derartiger Atemmasken Unannehmlichkeiten bereiten.

Dokument EP 1 099 452 A2 zeigt eine Atemmaske zur Zufuhr eines Atemgases zu einem Patienten sowie ein Verfahren zur Herstellung derselben. Die erfindungsgemäße Atemmaske umfasst eine nachgiebige Dichtungseinrichtung zur Abdichtung eines in einem Maskenbasiskörper gebildeten Maskeninnenraumes. Die Dichtungseinrichtung ist mit einem gelartig ausgebildeten Abschnitt versehen, der mit einer Hautschicht überzogen ist, die ebenfalls aus einem Elastomermaterial gebildet ist.

### Aufgabe der Erfindung

Der Erfindung liegt die Aufgabe zugrunde, ein Dichtkissen für eine Atemmaske sowie eine Atemmaske an sich zu schaffen, die sich durch eine hinreichend hohe Dichtwirkung und einen hohen Tragekomfort auszeichnet.

### Erfindungsgemäße Lösung

Diese Aufgabe wird gemäß einem ersten Aspekt der vorliegenden Erfindung gelöst durch eine Maskenkisseneinrichtung nach Anspruch 1.

Dadurch wird es auf vorteilhafte Weise möglich, eine Maskenkisseneinrichtung zu schaffen, die sich durch eine besonders hohe Adaptionsfähigkeit an unterschiedlichste, individuelle Gesichtstekturen auszeichnet.

Gemäß einer besonders bevorzugten Ausführungsform der Erfindung ist die Maskenkisseneinrichtung derart ausgebildet, dass die Shore-Härte des Maskenkissens im Bereich der in Applikationsposition im Stirn- oder Nasenrücken-nahen Umfangszonenbereich niedriger ist, als im Wangen-, Oberlippen- oder Nasenflügeln-nahen Umfangszonenbereich.

Dadurch wird es auf vorteilhafte Weise möglich, insbesondere im Bereich des Nasenrückens die Kisseneinrichtung wirkungsvoll und elastisch gepolstert im Stirn- oder Nasenrückenbereich abzustützen.

Vorzugsweise ist die Maskenkisseneinrichtung derart ausgebildet, dass diese im Bereich der querschnittsverdickten Zonen der Werkstoff gelartige Materialeigenschaften aufweist.

Die unterschiedlichen Werkstoffeigenschaften im Bereich der querschnittsverdickten Zonen können gemäß einer besonders bevorzugten Ausführungsform der Erfindung dadurch erreicht werden, dass die Aushärtung des Maskenkissen-Werkstoffes so erfolgt, dass der Werkstoff zonenweise unterschiedliche Werkstoff-Vernetzungsgrade aufweist.

Die genannten querschnittsverdickten Zonen können in der Maskenkisseneinrichtung derart ausgebildet sein, dass diese unmittelbar an die Dichtlippe angrenzen. Dadurch wird es möglich, die Funktion der Dichtlippe im wesentlichen auf die Herbeiführung einer hinreichenden Dichtwirkung zu beschränken und über die querschnittsverdickten Zonen die Atemmaskenanordnung auf der Gesichtsfläche des Maskenanwenders abzustützen.

Alternativ zu der vorangehend beschriebenen Maßnahme - oder bei zonenweise unterschiedlicher Ausgestaltung der Radial-Schnittgeometrie der Maskenkisseneinrichtung - ist es auch möglich, die querschnittsverdickten Zonen so auszubilden, dass diese auf der Gesichtsfläche des Maskenanwenders oder auf einer der Gesichtsfläche des Maskenanwenders abgewandten Innenseite der Dichtlippe aufsitzen.

Die unterschiedlichen Shore-Härten oder Elastizitäts-Module der Maskenkisseneinrichtung, insbesondere im Bereich der querschnittsverdickten Zonen können auch dadurch herbeigeführt werden, dass zur Bildung der Maskenkisseneinrichtung unterschiedlich vorbereitetes Elastomer-Compound-Material verwendet wird. Dieses unterschiedliche Elastomer-Compound-Material kann durch separate Speiseöffnungen in einen entsprechenden Formraum eines Formwerkzeuges eingebracht werden. Die Einspeisung der unterschiedlich vorbereiteten Elastomer-Materialien kann in zeitlich abfolgenden Schritten erfolgen.

Die erfindungsgemäße Maskenkisseneinrichtung kann derart ausgebildet sein, dass diese beispielsweise über einen Umfangsrandabschnitt in abdichtender Weise an einen als Hartschale ausgebildeten Gewölbekörper ansetzbar ist. Dadurch wird es möglich, die Maskenkisseneinrichtung zu Reinigungs- oder Ersatzzwecken von der Hartschale abzunehmen.

Alternativ zu der vorangehend beschriebenen Maßnahme ist es auch möglich, die Maskenkisseneinrichtung integral mit dem Gewölbekörper auszubilden. Hierbei wird die Ausbildung eines Spaltbereiches zwischen der Maskenkisseneinrichtung und dem Gewölbekörper vermieden.

Eine besonders hohe Adaptionsfähigkeit der erfindungsgemäßen Maskenkisseneinrichtung kann dadurch erreicht werden, dass die querschnittsverdickten Zonen zumindest abschnittsweise in Applikationsrichtung federnd aufgehängt sind. Diese federnde Aufhängung der querschnittsverdickten Zonen kann insbesondere über eine Balgstruktur, die beispielsweise als Falten- oder Rollbalg ausgebildet sein kann, erreicht werden. Es ist auch möglich, die Gesichtsdichtlippe über eine Falten- oder Rollbalgstruktur mit den querschnittsverdickten Zonen zu verbinden.

Die Maskenkisseneinrichtung kann so ausgebildet sein, dass die Radial-Querschnitte d.h. die Querschnitte der Maskenkisseneinrichtung variieren.

Die vorangehend beschriebene Maskenkisseneinrichtung bildet Bestandteil einer Atemmaske, die in Applikationsposition den Nasen- und/oder den Mundbereich eines Maskenanwenders übergreift. Sie kann in entsprechender Ausbildung bei einer Nasal-Maske oder auch bei einer Mund- oder Vollgesichtsmaske Anwendung finden.

Die erfindungsgemäße Gestaltung des Querschnitts der Elastomerstrukturen kann auch bei einem Stirnauflageelement Anwendung finden. So ist es gemäß einem weiteren, auch alternativen Lösungsgedanken möglich, Stirnauflage-Pads derart auszubilden, daß deren Verformungsverhalten durch Elastomerzonen verminderter Shore-Härte, oder durch Zonen erhöhten Porenvolumens geprägt ist.

Hinsichtlich eines Verfahren zur Herstellung der erfindungsgemäßen Maskenkisseneinrichtung wird die eingangs angegebene Aufgabe gelöst, durch ein Verfahren bei welchem im Rahmen eines Elastomer-Material-Einbringschrittes das Elastomermaterial in einen Dichtkissenformraum eingebracht wird, wobei die Temperaturverteilung der Formrauminnenwand sowie die Formschliesszeit derart abgestimmt werden, dass das in dem Formraum zu der Dichtkisseneinrichtung vernetzende Elastomermaterial unterschiedliche Shore-Härten erhält.

Es ist möglich, den Vernetzungsvorgang definiert abzubremsen, indem dem Elastomermaterial ein Katalyse-Blocker zugesetzt wird, der ab einem vorgegebenen Vernetzungsgrad, oder Vernetzungszeitraum, eine weitere Vernetzung unterbindet. Der Katalyse-Blocker ist vorzugsweise derart konfiguriert, daß dieser z.B durch UV-Belichtung, oder durch Mikrowellenaufheizung aktiviert wird. Durch die Verwendung eines Katalyse- oder Vernetzungs-Blockers wird es möglich, im Bereich jener Zonen mit niedrigem Vernetzungsgrad, diesen Zustand über einen hinreichend langen Zeitraum - Insbesondere auch unbeschadet einer Nachtemperphase - aufrecht zu erhalten.

Vorzugsweise wird hierbei das Temperaturprofil der Formrauminnenwand derart abgestimmt, dass die in dem Formraum gebildete Dichtkisseneinrichtung in ihrem Verlauf in Umfangsrichtung unterschiedliche Shore-Härten aufweist.

Das Temperaturprofil der Formrauminnenwand wird in vorteilhafter Weise weiterhin derart abgestimmt, dass die Maskenkisseneinrichtung in einem in Applikationsposition dem Stirn- oder Nasenrücken-nahen Bereichen niedrigere Shore-Härten erhält.

Es ist möglich, das teilweise unvernetzte Elastomermaterial abzusaugen oder auszublasen und das Maskenkissen nachzutempern. Auf diese Weise wird es möglich, in der Dichtkisseneinrichtung Kavernen- oder Schlauchzonen auszubilden.

In verfahrenstechnisch weiterhin besonders vorteilhafter Weise wird das Temperaturprofil der Formrauminnenwand während des Elastomermaterial-Einbringschnittes derart variiert, dass dieses zunächst eine erste gegebenenfalls weitgehend konstante Temperaturverteilung aufweist, wobei während der Formschliesszeit die Temperaturverteilung derart verändert wird, dass in ausgewählten Formraumzonen niedrigere Vernetzungsgrade und damit geringere Shore-Härten erreicht werden.

Insbesondere ist es möglich, die Temperaturverteilung des Formwerkzeuges derart abzustimmen, dass das Temperaturprofil während des Elastomer-Material-Einbringschrittes, und über eine sich daran anschließende Haltezeit eine erste Hochtemperaturverteilung hat, wobei nach Ablauf dieser Haltezeit ausgewählte Zonen der Formrauminnenwandung auf niedrigere Temperaturen abgekühlt werden.

Es wird auch ein Formwerkzeug zur Herstellung einer Maskenkisseneinrichtung beschrieben, das in Werkzeugschliessstellung einen durch eine Formrauminnenwandung begrenzten, zu der zu bildenden Dichtkisseneinrichtung komplementären Forminnenraum, und eine Heizeinrichtung zur Aufheizung der Formrauminnenwandung aufweist, wobei das Formwerkzeug derart ausgebildet ist, dass sich an der Formrauminnenwandung für unterschiedliche Zonen der darin zu bildenden Maskenkisseneinrichtung ein vorbestimmtes Temperaturprofil ergibt, das zu unterschiedlich hohen Vernetzungsgraden des in dem Forminnenraum aushärtenden Elastomermateriales führt.

Das Formwerkzeug ist vorzugsweise derart ausgebildet, dass das Temperaturprofil derart einstellbar ist, dass die Formraumtemperatur in einem zur Ausbildung des, den Stirn- oder Nasenrückenbereich abdichtenden Abschnitts der Dichtkisseneinrichtung, niedriger ist als die Temperatur in einem zur Ausbildung eines Oberlippen- oder Kinn-Dichtbereiches der Dichtkisseneinrichtung.

Eine im Hinblick auf eine besonders günstige Temperaturverteilung der Formrauminnenwandung vorteilhafte Ausführungsform des Formwerkzeuges, ist dadurch gegeben, dass das Formwerkzeug mit Kühlkanälen versehen ist, zur Abkühlung ausgewählter Abschnitte des Formwerkzeuges beispielsweise über ein zeitgesteuert eingebrachtes kühlmedium.

Es wird auch eine Dichtungsstruktur und ein Verfahren zur Herstellung derselben beschrieben. Insbesondere werden Dichtungsstrukturen zur abdichtenden Überbrückung oder Abdichtung, eines Spaltbereiches beispielsweise bei Rohr-Verbindungen, Gehäuseeinrichtungen, sowie Tür- und Fensteranordnungen beschrieben.

Üblicherweise ist bei derartigen Dichtungsstrukturen ein Dichtflächenabschnitt vorgesehen, der aus einem Elastomer-Material gefertigt ist und in elastisch nachgiebiger Weise an einer Anlagefläche aufsitzt.

Bei derartigen Dichtungsstrukturen besteht das Problem, dass die geforderte Dichtwirkung unter Umständen erst bei vergleichsweise hohen Flächenpressungen erreicht wird.

Die beschriebene Dichtungsstruktur ist unter fertigungstechnischen Gesichtspunkten günstig herstellbar und zeichnet sich durch eine hohe Dichtwirkung und hohe Adaptionsfähigkeit aus.

Die Dichtungsstruktur besteht aus einem aus einem Elastomer-Profilmaterial gefertigten Basiskörper, wobei der Basiskörper einen Profilquerschnitt mit wenigstens einer querschnittsverdickten Zone aufweist und das Elastomermaterial derart verarbeitet ist, dass die Shore-Härte des Profilmateriales im Randbereich der querschnittsverdickten Zone höher ist, als im Kern oder kernnahen Bereich der querschnittsverdickten Zone.

Dadurch wird es auf vorteilhafte Weise möglich, eine Dichtungsstruktur zu schaffen, die sich auch im Bereich der querschnittsverdickten Zone durch eine hohe Adaptionsfähigkeit auszeichnet.

Gemäß einer besonders bevorzugten Ausführungsform ist die Dichtungsstruktur derart ausgebildet, dass diese im Bereich der querschnittsverdickten Zonen gel-artige Materialeigenschaften aufweist. Die unterschiedlichen Werkstoffeigenschaften im Bereich der querschnittsverdickten Zone, können gemäß einer besonders bevorzugten Ausführungsform der Erfindung dadurch erreicht werden, dass die Aushärtung des Profilmateriales derart erfolgt, dass das Profilmaterial zonenweise unterschiedliche Werkstoff-Vernetzungsgrade aufweist.

Der Profilquerschnitt der Dichtungsstruktur kann derart gestaltet sein, dass die querschnittsverdickte Zone unmittelbar an eine integrale Dichtlippe angrenzt.

Die unterschiedlichen Shore-Härten oder Elastizitätsmodule des Profilmateriales, insbesondere im Bereich der querschnittsverdickten Zone, können dadurch herbeigeführt werden, dass die Dichtungsstruktur aus unterschiedlich vorbereiteten Elastomer-Mischungen (Compound-Material) gefertigt wird. Diese unterschiedlichen Elastomer-Compound Systeme können durch seperate Speiseöffnungen in einen Formraum eines Formwerkzeuges, insbesondere in einen Extrusionskanal eingebracht werden. Die Einspeisung der unterschiedlich vorbereiteten Elastomer-Compoundmischungen kann in zeitlich abfolgenden Schritten erfolgen.

Die Dichtungsstruktur kann derart ausgebildet sein, dass diese beispielsweise als umlaufender Dichtring bei einer Rohr-Verbindung vorgesehen ist.

Hinsichtlich eines Verfahrens zur Herstellung der Dichtungsstruktur, wird die eingangs angegebene Aufgabe gelöst, durch ein Verfahren, bei welchem im Rahmen eines Elastomer-Materialeinbringschrittes das ElastomerMaterial in einen Dichtungsstruktur-Formraum eingebracht wird, wobei die Aufheizung des Elastomer-Materiales in dem Formraum derart abgestimmt erfolgt, dass das Profilmaterial im Bereich einer querschnittsverdickten Zone einen niedrigeren Vernetzungsgrad erhält, als im Randbereich der querschnittsverdickten Zone oder verbleibenden dünnwandigen Abschnitten der Dichtungsstruktur.

Es ist möglich, den Vernetzungsvorgang definiert abzubremsen, indem dem Profilmaterial ein Katalyse-Blocker zugesetzt wird, der ab einem vorgegebene Vernetzungsgrad oder Vernetzungszeitraum eine weitere Vernetzung unterbindet. Dieser Katalyse-Blocker ist vorzugsweise derart konfiguriert, dass dieser zum Beispiel durch UV-Belichtung oder durch Mikrowellenaufheizung aktiviert wird. Durch die Verwendung eines Katalyse- oder Vernetzungs-Blocker wird es möglich, im Bereich jener Zonen mit niedrigem Vernetzungsgrad diesen Zustand über einen hinreichend langen Zeitraum, insbesondere auch unbeschadet einer Nachtemperphase aufrechtzuerhalten.

Vorzugsweise wird das Temperaturprofil einer zur Bildung der Dichtungsstruktur vorgesehenen Formrauminnenwand derart abgestimmt, dass die in dem Formraum gebildete Dichtungsstruktur Abschnitte unterschiedlicher Shore-Härte aufweist.

Es ist möglich, das im Bereich der querschnittsverdickten Zone gegebenenfalls unvernetzte Elastomer-Material zumindest teilweise abzusaugen oder auszublasen und die Dichtungsstruktur anschließend nachzutempern. Auf diese Weise wird es möglich, in der Dichtungsstruktur Kavernen- oder Schlauchzonen auszubilden.

Weitere Einzelheiten und Merkmale der angegebenen Erfindungen ergeben sich aus der nachfolgenden Beschreibung in Verbindung mit der Zeichnung.

### Kurzbeschreibung der Figuren

Es zeigt:
- **Fig. 1**: einen Querschnitt durch eine Dichtkisseneinrichtung gemäß einer ersten Ausführungsform der Erfindung mit einer querschnittsverdickten Zone und darin enthaltenen Bereichen unterschiedlicher Shore-Härte;
- **Fig. 2**: eine Schnittansicht durch eine Dichtkisseneinrichtung ebenfalls mit einer querschnittsverdickten Zone und darin ausgebildeten Bereichen unterschiedlicher Materialeigenschaften;
- **Fig. 3**: ein Diagramm zur Erläuterung der unterschiedlichen Temperaturverteilung einer Formrauminnenwand eines Formwerkzeuges zur Herstellung einer Dichtkisseneinrichtung mit in Umfangsrichtung variierender Shore-Härte.
- **Fig.4**: eine Schnittansicht zur Erläuterung des Querschnitts einer Dichtkisseneinrichtung mit einem, in deren Innenbereich, einer Dichtlippe benachbart angeordneten, gelartig vernetzten Einsatzelement.
- **Fig. 5a**: eine Variante des Einsatzelementes 16 nach welcher deren gelartig vernetzter Körper eine hufeisenartige Gestalt aufweist und sich in Applikationsposition über den Nasenrücken des Maskenanwenders erstreckt.
- **Fig.5b**: zeigt eine Variante des Einsatzelementes 16 nach welcher deren gelartig vernetzte Körper ringartig der Dichtlippe folgend, um eine Mund- und/oder Nasenöffnung umlaufend ausgebildet ist;
- **Fig. 6a**: eine Schnittansicht durch eine Dichtkisseneinrichtung ebenfalls mit einer querschnittsverdickten und einem Gel- oder Schaummaterial ausgefüllten Zone die hier durch einen integral mit der Dichtlippeneinrichtung gebildeten Taschenabschnitt gebildet ist, wobei der Taschenabschnitt durch ein Rahmenelement abgedeckt ist und die Dichtlippeneinrichtung mit einem Schalenkörper versehen ist, der integral mit der Dichtlippeneinrichtng aus einem Elastomermaterial gefertigt ist;
- **Fig.6b**: eine Schnittansicht durch eine Dichtkisseneinrichtung ähnlich Figur 6b, wobei das Rahmenelement eine Anschlussstruktur bildet, zur Ankoppelung einer den Nasenbereich überdeckenden Maskenschale;
- **Fig. 6c**: eine weitere Schnittansicht durch eine Dichtkisseneinrichtung ebenfalls mit einer querschnittsverdickten und mit einem nachgiebigen Gel- oder Schaummaterial gefüllten querschnittsverdickten Zone;
- **Fig. 7**: eine vereinfachte perspektivische Darstellung eines aus einem Gel- oder Schaummaterial bestehenden Polsterkörpers zur Abstützung einer Atmmaske;
- **Fig. 8**: eine vereinfachte Seitenansicht zur Erläuterung eines möglichen Verlaufs eines Polsterkörpers nach Fig. 7 auf der Gesichtsfläche eines Maskenanwenders;
- **Fig. 9**: eine vereinfachte perspektivische Darstellung eines Geloder Schaumpolsterkörpers, der in verschiedene Segmente unterteilt ist, wobei die Segmente unterschiedliche mechanische Eigenschaften, insbesondere unterschiedliche Shorehärten aufweisen und die Grenzflächen organisch uneben ausgebildet sind;
- **Fig. 10**: eine räumliche Darstellung zur Erläuterung einer Polsterkörpervariante mit einem im Nasenrückenüberbrückungsbereich ausgesparten Abschnitt;
- **Fig. 11**: eine Schnittansicht eines den Nasenrückenbereich überbrückenden Polsterkörpersegmentes;
- **Fig. 12**: eine Skizze zur Illustration des Verlaufs eines Polsterkörpers auf der Gesichtsfläche eines Patienten;
- **Fig. 13**: eine Skizze zur Erläuterung eines auf dem Oberlippenbereich eines Patienten aufsitzenden Polsterkörpersegments;
- **Fig. 14**: eine perspektivische Ansicht einer Dichtlippeneinrichtung einschließlich zugehörigem Maskenbasisköpers, wobei die Dichtlippeneinrichtung einen Umfangstaschenabschnitt aufweist der mit einem Gel- oder Schaumstoffmaterial gefüllt ist und im Bereich der abgesetzt gekennzeichneten Dichtlippenumfangszone den Maskenbasiskörper auf der Gesichtsfläche des Patienten abstützt;
- **Fig. 15**: eine perspektivische Ansicht einer Dichtlippeneinrichtung ähnlich Figur 14 einschließlich zugehörigem Maskenbasisköpers, wobei die Dichtlippeneinrichtung einen Umfangstaschenabschnitt aufweist der mit einem Gel- oder Schaumstoffmaterial gefüllt ist und im Bereich der abgesetzt gekennzeichneten Dichtlippenumfangszone den Maskenbasiskörper auf der Gesichtsfläche des Patienten abstützt und im Berich des Nasenrückens mit einer Ausnehmung versehen ist, oder unterbrochen ist, so dass in dieser Zone keine Abstützung über das Gel- oder Schaumstoffmaterial auf dem Nasenrücken erfolgt;
- **Fig. 16**: eine perspektivische Ansicht der Dichtlippeneinrichtung nach Figur 15 zur Illustration des partiell unterbrochenen Umfangstaschenabschnitts;
- **Fig. 17**: eine perspektivische Ansicht einer Atemmaske mit schwenkbewegbar angelenkter Stirnauflageeinrichtung und einer Dichtlippeneinrichtung einschließlich zugehörigem Maskenbasisköpers, gemäß Figuren 14 oder 15;
- **Fig. 18a bis 18 e**: Schnittansichten von Varianten von Strinauflage-Pads insbesondere zur Verwendung in Verbindung mit einer Atemmaske, beispielsweise gemäß Figur 17, wobei die Stirnauflagepads Querschnittsverdickte Zonen aufweisen die mit einem Gel- oder Schaumstoffmaterial gefüllt sind;
- **Fig. 19**: eine perspektivische Ansicht eines Abschnitts einer erfindungsgemäßen Dichtungsstruktur mit einer querschnittsverdickten Zone und darin ausgebildeten Bereichen niedriger Shore-Härte.

### Ausführliche Beschreibung der Figuren

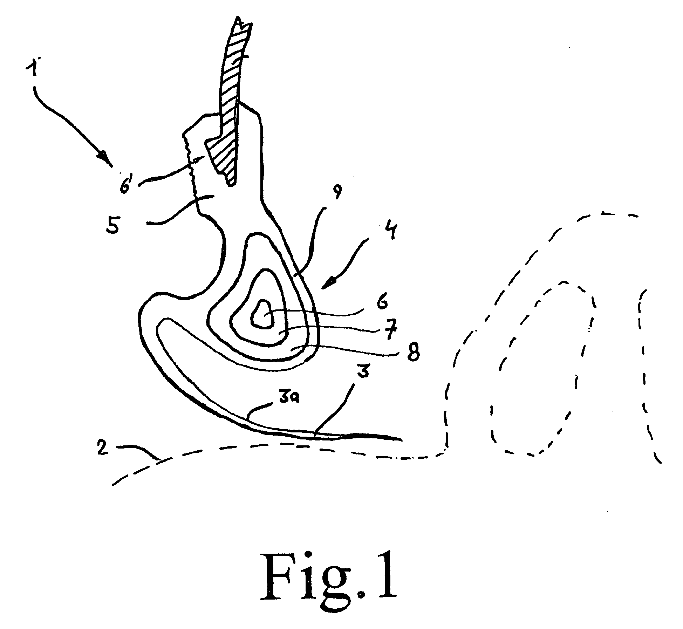

Die in Fig. 1 dargestellte Dichtkisseneinrichtung 1 umfasst eine in Applikationsposition auf einer hier angedeuteten Gesichtsfläche 2 aufsitzenden Dichtlippe 3.

Die Maskenkisseneinrichtung 1 umfasst weiterhin eine querschnittsverdickte Zone 4, die bei diesem Ausführungsbeispiel zwischen einem oberen Umfangsrand 5 und der Dichtlippe 3 angeordnet ist.

Die querschnittsverdickte Zone 4 ist derart ausgebildet, dass die Shore-Härte des die querschnittsverdickte Zone bildenden Materiales, variiert. Die hier angedeuteten Materialzonen haben einen nahezu gelartigen Charakter. Die Shore-Härte der am weitesten innen liegenden Materialzone 6 ist niedriger, als die der angrenzenden Materialzone 7, welche wiederum eine niedrigere Shore-Härte hat, als die angrenzende Materialzone 8. Der Außenbereich 9 der querschnittsverdickten Zone ist aus einem im wesentlichen vollständig vernetzten Elastomermaterial gefertigt und hat im wesentlichen dieselbe Shore-Härte wie die Dichtlippe 3.

Die unterschiedlichen Materialeigenschaften im Bereich der querschnittsverdickten Zone 4 werden durch das Temperaturprofil eines zur Fertigung der Maskenkisseneinrichtung 1 vorgesehenen Formwerkzeuges sowie durch Begrenzung der Verweilzeit, der zumindest im Formraum-nahen Bereich, hinreichend ausgehärteten Maskeneinrichtung in dem Formwerkzeug bestimmt.

Es ist möglich, den Vernetzungsvorgang definiert abzubremsen, indem dem Elastomermaterial ein Katalyse-Blocker zugesetzt wird, der ab einem vorgegebenen Vernetzungsgrad, oder Vernetzungszeitraum, eine weitere Vernetzung unterbindet. Der Katalyse-Blocker ist vorzugsweise derart konfiguriert, daß dieser z.B. durch UV-Belichtung, oder durch Mikrowellenaufheizung aktiviert wird. Durch die Verwendung eines Katalyse- oder Vernetzungs-Blockers wird es möglich, im Bereich jener Zonen mit niedrigem Vernetzungsgrad, diesen Zustand über einen hinreichend langen Zeitraum - Insbesondere auch unbeschadet einer Nachtemperphase - aufrecht zu erhalten.

Bei der hier dargestellten Maskenkisseneinrichtung 1 ist die Dichtlippe 3 derart mit der querschnittsverdickten Zone 4 gekoppelt, dass die querschnittsverdickte Zone 4 in Applikationsposition der Maskenkisseneinrichtung 1 gegebenenfalls auf einer Innenseite 3a der Dichtlippe 3 aufsitzen kann. Hierdurch kann über die querschnittsverdickte Zone 4 die Maskenkisseneinrichtung 1 auf der Gesichtsfläche 2 abgestützt werden, wobei sich auf Grund der besonderen Eigenschaften der querschnittsverdickten Zone 4, nur vergleichsweise geringe Flächenpressungen ergeben.

Vermittels der querschnittsverdickten Zone 4 ist es bei dieser Ausführungsform auch möglich, die Dichtlippe 3 insbesondere in den kritischen Bereichen wie zum Beispiel dem Nasenrückenbereich zusätzlich gegen die Gesichtsfläche des Maskenanwenders zu drängen.

Im Bereich des oberen Umfangsrandes 5 ist hier eine Koppelungsstruktur 6' ausgebildet, über welche die Maskenkisseneinrichtung 1 mit einer hier nur andeutungsweise dargestellten, eine Gewölbekörper bildenden Hartschale in hinreichend abdichtender Weise gekoppelt werden kann.

Die hier dargestellte Maskenkisseneinrichtung 1 ist aus einem Zwei-Komponenten-Silikonmaterial gefertigt, wobei die Materialzone 6 im wesentlichen nicht ausgehärtet ist. Die Materialzonen 7 und 8 sind gelartig-teilausgehärtet, wobei der Vernetzungsgrad der Materialzone 8 größer ist als der Vernetzungsgrad in der Materialzone 7.

Die höheren Vernetzungsgrade im Randbereich der Maskenkisseneinrichtung 1 werden insbesondere durch die hohe Aufheizung des an eine Formraumwandung angrenzenden Silikonmateriales erreicht.

Unabhängig von der vorangehend beschriebenen Ausgestaltung der querschnittsverdickten Zone 4 - oder in besonders vorteilhafter Weise in Kombination mit dieser - ist es möglich, die Dichtlippe 3 derart auszubilden, dass diese in ihrem Umfangsverlauf unterschiedliche Shore-Härten aufweist. Diese unterschiedlichen Shore-Härten können ebenfalls durch willkürliche Festlegung des Temperaturprofiles eines zur Bildung der Maskenkisseneinrichtung 1 vorgesehenen Formwerkzeuges im Bereich seiner Formrauminnenwandung erreicht werden.

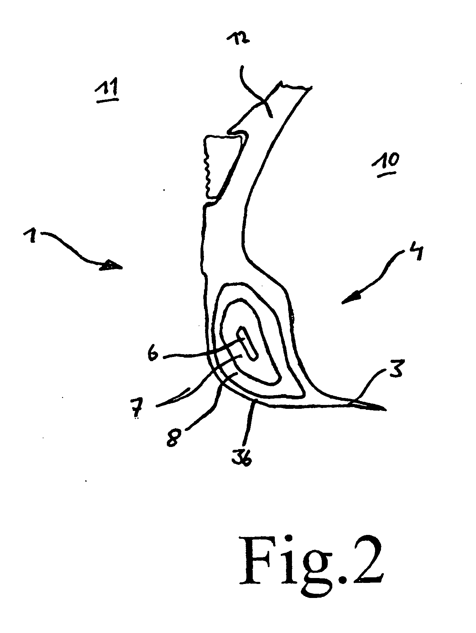

In Fig. 2 ist ein weiteres Ausführungsbeispiel einer Maskenkisseneinrichtung 1 dargestellt, die hier integral mit einem den Maskeninnenraum 10 vom Umgebungsbereich 11 trennenden Gewölbekörper 12 ausgebildet ist.

Die auch bei dieser zweiten Ausführungsform vorgesehene querschnittsverdickte Zone 4 weist ebenfalls Materialzonen 6, 7, 8 auf, die sich hinsichtlich der darin vorherrschenden Shore-Härte unterscheiden. Die querschnittsverdickte Zone sitzt bei diesem Ausführungsbeispiel über eine vollständig vernetzte, und in die Dichtlippe 3 übergehende Aussenwandung 3b auf der hier näher dargestellten Gesichtsfläche des Maskenanwenders ab.

Die Dichtlippe 3 ist hier nur als kleiner, zur Nasen- und/oder Mundöffnung radial einwärts vordringender Dichtlippenabschnitt ausgebildet.

Obgleich hier nicht näher dargestellt, ist es möglich, die querschnittsverdickte Zone 4 und/oder die Dichtlippe 3 über Faltenstrukturen mit dem Gewölbekörper 12 zu koppeln, so dass hierdurch eine nochmals verbesserte Adaptionsfähigkeit der Maskenkisseneinrichtung 1 an die individuelle Gesichtstektur des Maskenanwenders ergibt.

Die querschnittsverdickte Zone 4 kann mit gegebenenfalls variierender Querschnittsgeometrie vollständig umlaufend ausgebildet sein. Alternativ hierzu ist es jedoch auch möglich, die querschnittsverdickte Zone in der Maskenkisseneinrichtung 1 nur im Bereich des, in Applikationsposition dem Stirn- oder Nasenrücken-nahen Bereich auszubilden.

Es ist auch möglich, in Umfangsrichtung der Maskenkisseneinrichtung 1 derart unterschiedliche Radial-Schnittgeometrien vorzusehen, dass beispielsweise im Oberlippen- oder Kinn-nahen Bereich der Querschnitt durch die Maskenkisseneinrichtung 1 im wesentlichen dem in Fig. 2 skizzierten Aufbau entspricht, wogegen im Stirn- oder Nasenrücken-nahen Bereich der Maskenkisseneinrichtung 1 der Querschnitt der Maskenkisseneinrichtung 1 im wesentlichen dem in Fig. 1 dargestellten Querschnitt entspricht.

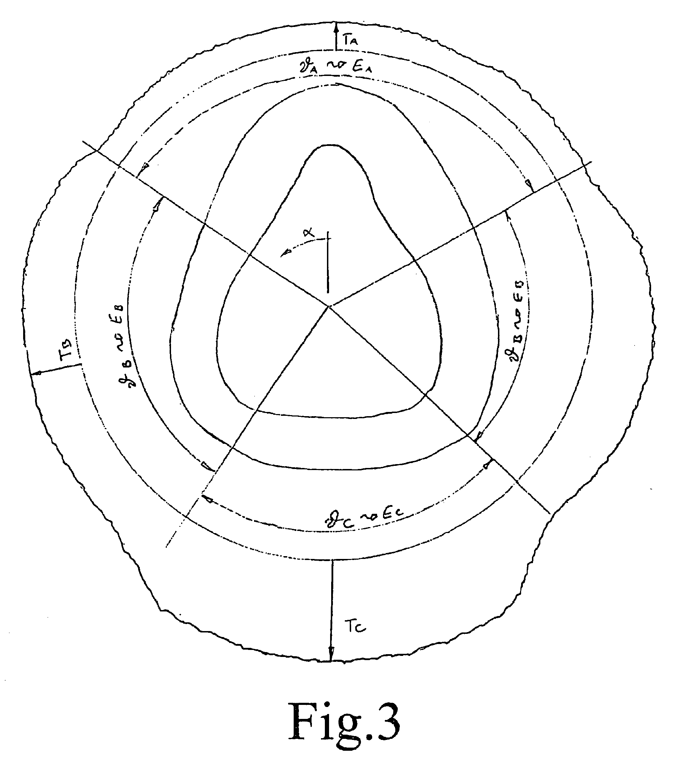

In Fig. 3 ist in der Art eines Polardiagrammes der Temperaturverlauf einer, der Dichtlippeneinrichtung 3 sowie insbesondere der querschnittsverdickten Zone 4 benachbarten Formrauminnenwandung dargestellt. Wie aus diesem Polardiagramm ersichtlich, herrscht in einem hier als □ A gekennzeichneten Polarbereich eine vergleichsweise niedrige Durchschnittstemperatur TA, die zu einem niedrigeren Vernetzungsgrad des Elastomerwerkstoffes der Maskenkisseneinrichtung 1 führt, als die Temperaturen TB und TC in den Bereichen □ B und □ C.

Die Temperatur TC im Bereich ϑC übersteigt die Temperatur TB im Bereich ϑB.

Die Temperatur im Bereich ϑA bestimmt im wesentlichen den Vernetzungsgrad und damit die Shore-Härte der Dichtlippe 3 in einem Stirn- oder Nasenrücken-nahen Bereich.

Die Temperatur TB in den Bereichen ϑB bestimmt im wesentlichen die Shore-Härte in den Wangen oder Nasenflügel-nahen Bereich der Dichtlippe 3. Die Temperatur TC bestimmt im wesentlichen die Shore-Härte der Dichtlippe 3 in ihrem auf dem Kinn- oder Oberlippenbereich des Maskenanwenders aufsitzenden Umfangsabschnitt.

Die hier dargestellte Temperaturverteilung kann während der Verweilzeit des Elastomermateriales in dem entsprechenden Formraum eines Formwerkzeuges verändert werden. Die Änderung des Temperaturprofiles in dem Formraum des Formwerkzeuges kann durch Änderung der Heizleistung oder durch abschnittsweise Kühlung des Formwerkzeuges erfolgen.

Kühl- und/oder Heizorgane können sowohl im Bereich der Aussenwerkzeuge als auch im Bereich der in Schließstellung des Formwerkzeuges in den Außenwerkzeugen unter Belassung des Dichtkissen-Formraumes aufgenommenen Formkernwerkzeugen ausgebildet sein.

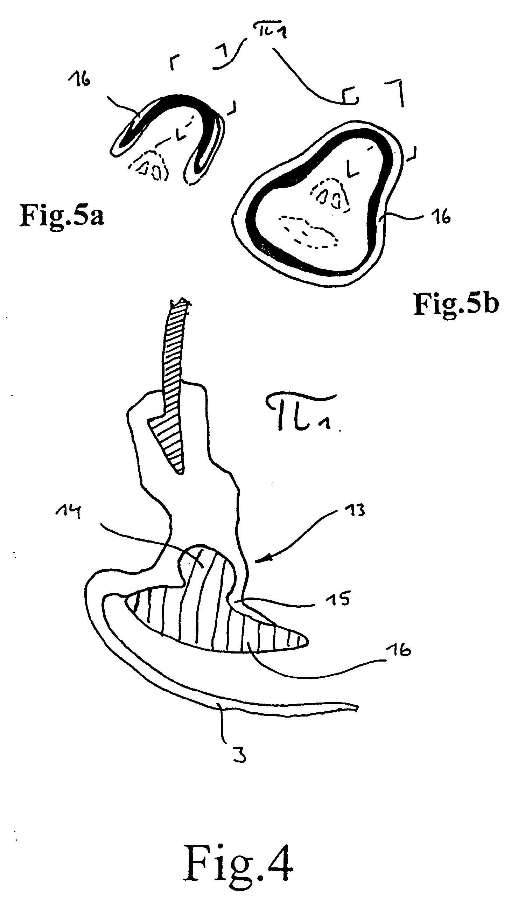

Die in Figur 4 dargestellte Dichtkisseneinrichtung umfaßt ein Einsatzelement 16 das aus einem gelartig ausgehärteten, oberflächlich verhauteten Elastomermaterial gefertigt ist. Dieses Einsatzelement ist über eine Fixierstruktur 13 im Innenbereich der Dichtkisseneinrichtung angeordnet. Die Fixierstruktur 13 ist durch eine Profilierung der Dichtkisseneinrichtung integral mit dieser ausgebildet. Die Profilierung ist derart gestaltet, daß ein Wulstabschnitt 14 des Einsatzelementes 16 durch einen Haltelippenabschnitt 15 gehalten ist. Ein der Dichtlippe 3 zugewandter Abschnitt des Einsatzelementes 16 ist derart ausgebildet, daß dieser ggf. auf einer Innenseite der Dichtlippe 3 aufsitzen kann.

Es ist möglich, die Fixerstruktur im Innenbereich der Dichtkisseneinrichtung derart auszubilden, daß an diese unterschiedlich ausgebildete Einsatzelemente ankoppelbar sind. Es ist möglich, mehrere, Dichtkissen-kompatible Einsatzelemente in Gel- Schaum- und/oder Schlauchbauform vorzusehen und diese anwendungsfallbezogen auszuwählen und eine Atemmaske damit zu bestücken. Es ist möglich, wenigstens eine Variante eines zumindest teilweise vorgeformten, vorzugsweise gelartigen, Einsatzelementes vorzuhalten, das beispielsweise durch Erwärmung in einen hinreichend plastifizierten Zustand bringbar ist, in welchem das Einsatzelement, an die individuelle Gesichtstektur des Maskenanwenders anpassbar ist.

Die Skizze Fig.5a zeigt eine Variante des Einsatzelementes 16 nach welcher deren gelartig vernetzter Körper eine hufeisenartige Gestalt aufweist und sich in Applikationsposition über den Nasenrücken des Maskenanwenders erstreckt.

Die Skizze Fig.5b zeigt eine Variante des Einsatzelementes 16 nach welcher deren gelartig vernetzte Körper ringartig der Dichtlippe folgend, um eine Mund- und/oder Nasenöffnung umlaufend ausgebildet ist.

Der Querschnitt des Einsatzelementes 16 im Bereich der Schnittebene π entspricht beispielsweise dem in Figur 4 dargestellten Querschnitt.

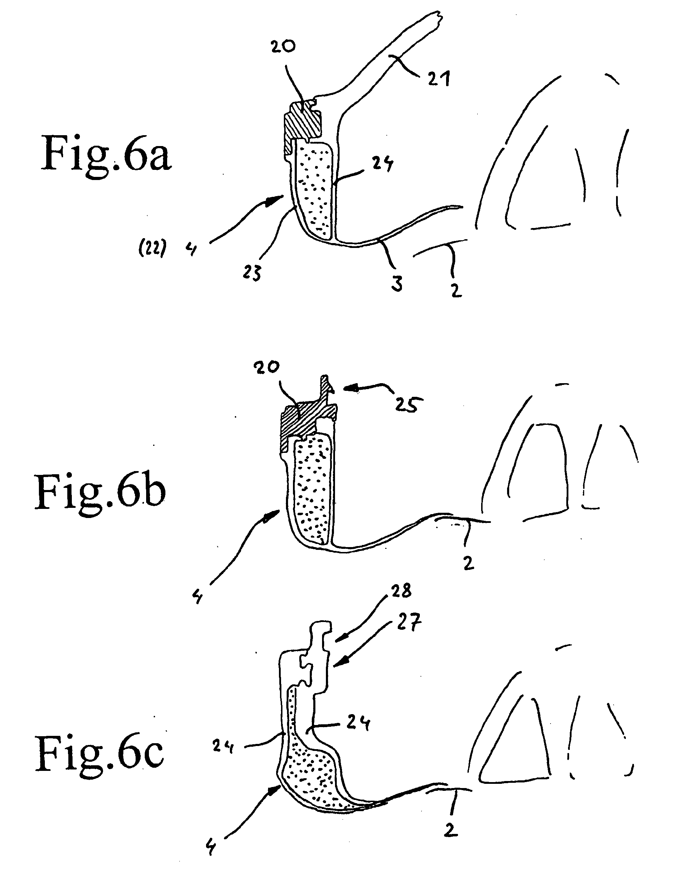

Figur 6a zeigt eine Schnittansicht durch eine Dichtkisseneinrichtung ebenfalls mit einer querschnittsverdickten und einem Gel- oder Schaummaterial ausgefüllten Zone die hier in einem integral mit der Dichtlippeneinrichtung gebildeten Taschenabschnitt 22 gebildet ist, wobei der Taschenabschnitt 22 durch ein Rahmenelement 20 abgedeckt ist und die Dichtlippeneinrichtung mit einem Schalenkörper 21 versehen ist, der integral mit der Dichtlippeneinrichtung, insbesondere der Dichtlippe 3 aus einem Elastomermaterial gefertigt ist. Der Taschenabschnitt 22 ist durch zwei Wandabschnitte 23, 24 definiert, die Teil der Dichtlippeneinrichtung bilden. Das Rahmenelement 20 bildet Teil einer Applikationsvorrichtung zur Applikation der Dichtkisseneinrichtung auf dem Gesicht eines Atemmaskenanwenders. An dem Rahmenelement sind Profilierungen und Raststrukturen vorgesehen, die eine definierte Koppelung der Dichtlippeneinrichtung mit dem Rahmenelement 20 ermöglichen. Das Rahmenelement kann aus einem biegsamen Material gefertigt sein, so dass eine weitere Anpassung des Maskenkissens an die individuelle Gesichtstektur eines Maskenanwenders ermöglicht ist. Bei dem gezeigten Ausführungsbeispiel bildet das Rahmenelement 20 eine Haltestruktur, durch welche der Taschenabschnitt 22 abgeschlossen wird. Das Rahmenelement 20 kann mit der Dichtlippeneinrichtung spaltfrei verklebt sein.

Figur 6b zeigt eine Schnittansicht durch eine Dichtkisseneinrichtung ähnlich Figur 6a, wobei das Rahmenelement 20 eine Anschlussstruktur 25 bildet, zur Ankoppelung einer den Nasenbereich überdeckenden, beispielsweise aus POM gefertigten Maskenschale. das Rahmelement 20 bildet auch bei diesem Ausführungsbeispiel eine Haltestruktur, durch welche der Taschenabschnitt 4 verschlossen ist.

Figur 6c zeigt eine weitere Schnittansicht durch eine Dichtkisseneinrichtung ebenfalls mit einer querschnittsverdickten und mit einem nachgiebigen Gel- oder Schaummaterial gefüllten querschnittsverdickten Zone 4. Die Dichtlippeneinrichtung umfasst ähnlich wie die Ausführungsform nach Figur 6a zwei Wandabschnitte 23, 24 zwischen welchen das Gel- oder Schaummaterial aufgenommen ist. Die Wandabschnitte 23, 24 weisen eine Verschlussprofilierung 27 auf und sind durch diese zusammengehalten. Die so gebildete Dichtlippeneinrichtung kann über einen Halterandabschnitt 28 an einer Hartschale einer Atemmaske angebracht werden;

In den Figuren 6a, 6b und 6c ist andeutungsweise der Nasenbereich eines Patienten dargestellt. Die Querschnittsgestaltung der Dichtlippeneinrichtung kann in ihrem Verlauf in Umfangsrichtung variieren und unterschiedliche Stützcharakteristika und Flächenpressungen erzeugen. Vorzugsweise liegt der Flächenanteil der unter Wirkung des Gel- oder Schaumstoffkörpers abgestützten Hautkontaktzone der Dichtlippeneinrichtung im Bereich von 18 bis 54 %. Der von der Gel- oder Polsterkörpergestützen Zone umsäumte Flächenanteil der Hautkontaktzone liegt vorzugsweise im Bereich von 46 bis 82%.

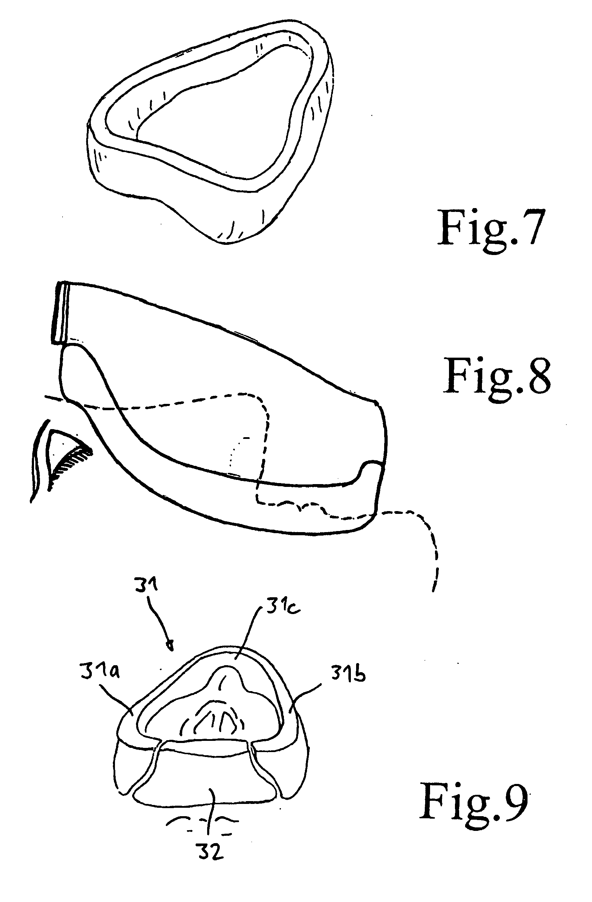

In Figur 7 ist eine vereinfachte perspektivische Darstellung eines aus einem Gel- oder Schaummaterial bestehenden profilierten Polsterkörpers zur Abstützung einer Atemmaske gezeigt, der in seinem Verlauf in Umfangsrichtung verschiedene Querschnitte bereitstellt. Der Polsterkörper ist derart ausgebildet, dass dieser im Bereich der zur Überbrückung des Nasenrückens vorgesehenen Zone nur eine relativ kleine Querschnittsfläche aufweist. In den zur Abstützung auf Backenknochen oder Wangenbereich vorgesehenen Zonen ist der Polsterkörper relativ dickwandig und stark verformbar ausgebildet.

Figur 8 zeigt eine vereinfachte Seitenansicht zur Erläuterung eines möglichen Verlaufs eines Polsterkörpers nach Figur 7 auf der Gesichtsfläche eines Maskenanwenders. Der Polsterkörper kann in einem hinreichend komplementär ausgebildeten Taschenabschnitt einer Dichtlippeneinrichtung aufgenommen sein, wie diese in Verbindung mit den Figuren 6a, 6b und 6c sowie den Figuren 14, 15, 16 beschrieben ist.

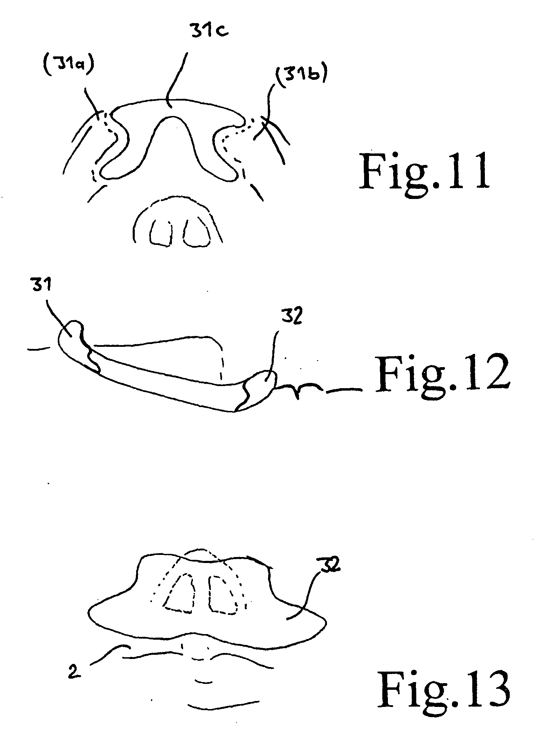

Figur 9 zeigt eine vereinfachte perspektivische Darstellung eines Gel- oder Schaumpolsterkörpers, der in verschiedene Segmente unterteilt ist, wobei die Segmente unterschiedliche mechanische Eigenschaften, insbesondere unterschiedliche Shorehärten aufweisen und die Grenzflächen organisch uneben ausgebildet sind. Bei dem gezeigten Ausführungsbeispiel ist der Polsterkörper aus einem Gelmaterial gefertigt und in ein Flankenstützsegment 31 und ein Oberlippenstützsegment 32 unterteilt. Die beiden Segmente sind aus unterschiedlich eingefärbten, transparenten Gelmaterialien gefertigt und in eingebautem Zustand in einer Umfangstasche einer Dichtlippeneinrichtung aufgenommen. Das Flankenstützsegment 31 weist einen linken Flankenabschnitt 31a und einen rechten Flankenabschnitt 31b auf, die hier im Nasenrückenbereich über eine Brückenzone 31c miteinander verbunden sind. Es ist auch möglich, im Bereich der Brückenzone ein von den Flankenabschnitten 31a, 31b separates, vorprofiliertes Segment vorzusehen, wie dies in Figur 11 dargestellt ist. es ist auch möglich, die Beiden Flankenabschnitte 31a, 31b im Beriech des Nasenrückens zu unterbrechen.

Die Segmente 31a, 31b, 31c, 32 sind vorzugsweise so ausgebildet, dass diese über uneben ausgebildete Grenzflächen aneinander stoßen. Der Verlauf der Grenzflächen kann unter ästhetischen sowie strukturmechanischen Gesichtspunkten abgestimmt werden und dabei insektenpanzerartige Segmentgrenzkonturen aufweisen.

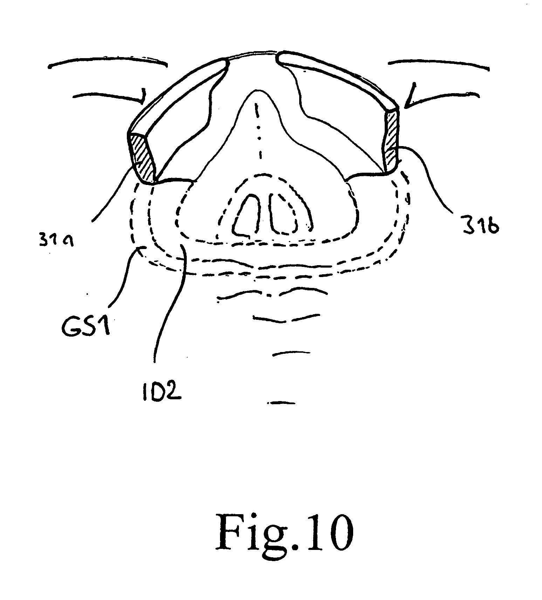

Figur 10 zeigt eine räumliche Darstellung zur Erläuterung einer Polsterkörpervariante mit einem im Nasenrückenüberbrückungsbereich ausgesparten Abschnitt. Die Flankenabschnitte 31a, 31b bilden Stützsysteme zur Übertragung einer Maskenstützkraft auf die Gel- oder Schaumkörperstützzone G/S1. Diese Gel- oder Schaumkörperstützzone G/S1 umsäumt eine Innenlippenauflagezone ID2 die sich über den Oberlippen oder Kinnbereich, sowie den Nasenrückenbereich erstreckt. Dieser Polsterkörper kann in einen Taschenabschnitt einer Dichtlippeneinrichtung eingesetzt sein.

Fig. 11 zeigt eine Schnittansicht eines den Nasenrückenbereich überbrückenden Polsterkörpersegmentes 31c das aus einem besonders weichen, schwachvernetztem Gelmaterial gefertigt ist und den Nasenrücken überquert. Das Polsterkörpersegment 31c ist so ausgebildet, dass dieses Anschlussflächen aufweist die einen mechanisch vorteilhaften Übergang zu den Seitenflankensegmenten 31a, 31b ermöglichten.

Fig. 12 zeigt eine Skizze zur Illustration des Übergangsbereiches zwischen einem Polsterkörpersegment 31c und einem Seitenflankensegment 31a, sowie insgesamte den Verlauf eines derartigen, aus mehreren Segmenten gebildeten Polsterkörpers auf der Gesichtsfläche eines Patienten. Das jeweils weichere Segment bildet einen Auslaufabschnitt der das härtere Segment untergreift.

Figur 13 zeigt eine Skizze zur Erläuterung eines auf dem Oberlippenbereich eines Patienten aufsitzenden Polsterkörpersegments 32 wie dies bei dem segmentierten Polsterkörper gemäß Figur 12 vorgesehen ist. Die Polsterkörpersegmente bestehen vorzugsweise aus einem Gel- oder Elastomermaterial mit niedrigem Vernetzungsgrad und sind als solche in komplementär ausgebildeten Taschenabschnitten unmittelbar aneinanderstoßend, oder durch Stege voneinander getrennt aufgenommen.

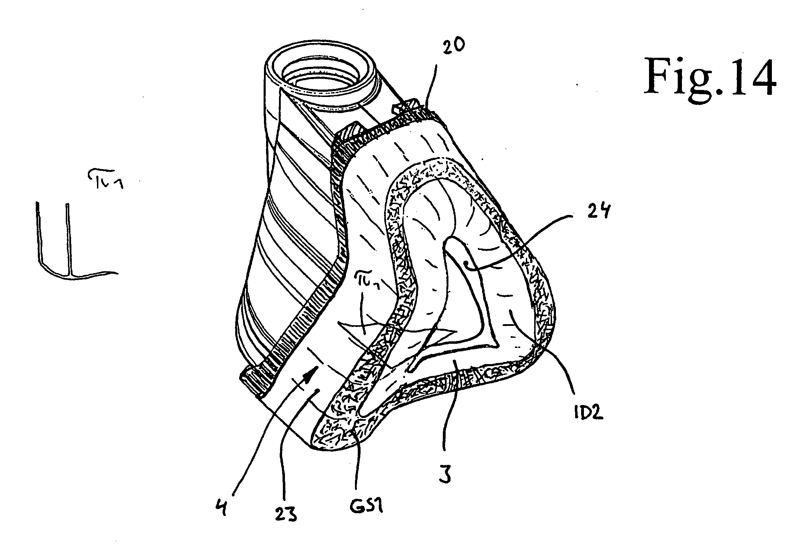

Figur 14 zeigt eine perspektivische Ansicht einer Dichtlippeneinrichtung einschließlich zugehörigem Maskenbasisköpers, wobei die Dichtlippeneinrichtung einen Umfangstaschenabschnitt 4 aufweist der zur Bereitstellung eines Polsterkörpers mit einem Gel- oder Schaumstoffmaterial gefüllt ist (siehe Figuren 6a, 6b nd 6c). Die derart ausgebildete Dichtlippeneinrichtung stützt im Bereich der durch Wirrschraffur abgesetzt gekennzeichneten Dichtlippenumfangszone den Maskenbasiskörper auf der Gesichtsfläche des Patienten in der Zone GS1 (siehe auch Fig. 10). Der Innenbereich der Dichtlippeneinrichtung 3 liegt von den Maskenanpresskräften weitgehend unabhängig über die Zone ID2 auf der Gesichtsfläche des Maskenanwenders auf.

Der Umfangstaschenabschnitt 4 ist von der Wandung 23 eingefasst. Die von der Wandung 23 umschlossene und im Zusammenspiel mit einer Innenumfangswandung 24 gebildete Tasche ist durch ein Rahmenelement 20 abgedeckt. Über das Rahmenelement 20 kann unmittelbar eine Aufbringung von Maskenanpresskräften auf das im Taschenabschnitt 4 aufgenommene Gel- oder Polstermaterial erfolgen.

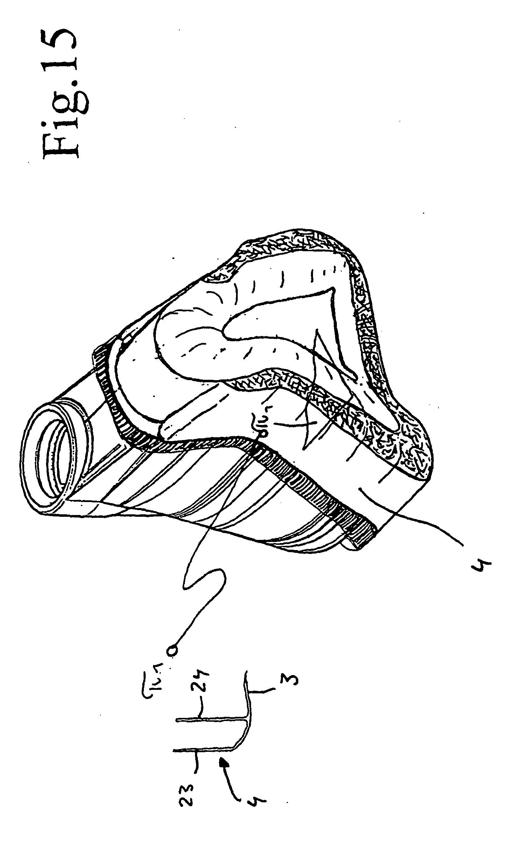

In Figur 15 ist eine perspektivische Ansicht einer Dichtlippeneinrichtung ähnlich Figur 14 einschließlich zugehörigem Maskenbasisköpers gezeigt, wobei die Dichtlippeneinrichtung einen Umfangstaschenabschnitt aufweist der mit einem Gel- oder Schaumstoffmaterial gefüllt ist und im Bereich der abgesetzt gekennzeichneten Dichtlippenumfangszone den Maskenbasiskörper auf der Gesichtsfläche des Patienten abstützt. Im Bereich des Nasenrückens ist der Polsterkörper mit einer Ausnehmung versehen oder unterbrochen, so dass in dieser Zone keine Abstützung über das Gel- oder Schaumstoffmaterial auf dem Nasenrücken erfolgt;

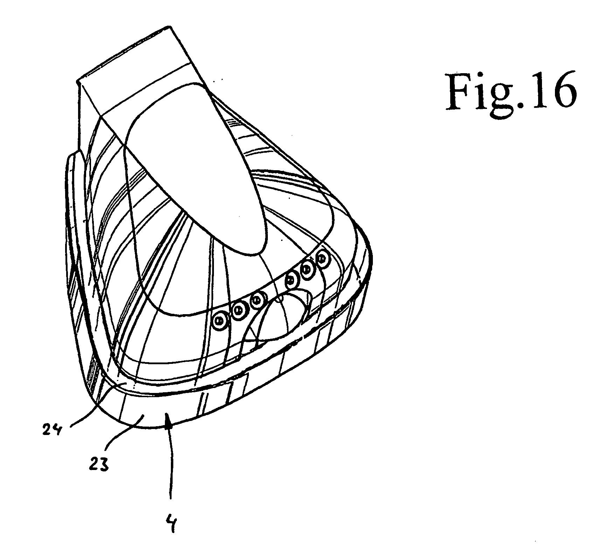

Figur 16 zeigt eine perspektivische Ansicht der Dichtlippeneinrichtung nach Figur 15 zur Illustration des Umfangstaschenabschnitts 4. In diesen Umfangstaschenabschnitt kann das zur Bildung des Polsterkörpers vorgesehene Material ungeformt eingebracht werden und in dem Taschenabschnitt definiert vernetzen. Es ist auch möglich, in den Taschenabschnitt vorgeformte Polsterkörper, insbesondere segmentierte Polsterkörper einzusetzen und darin zum Beispiel durch ein Rahmenelement zu sichern.

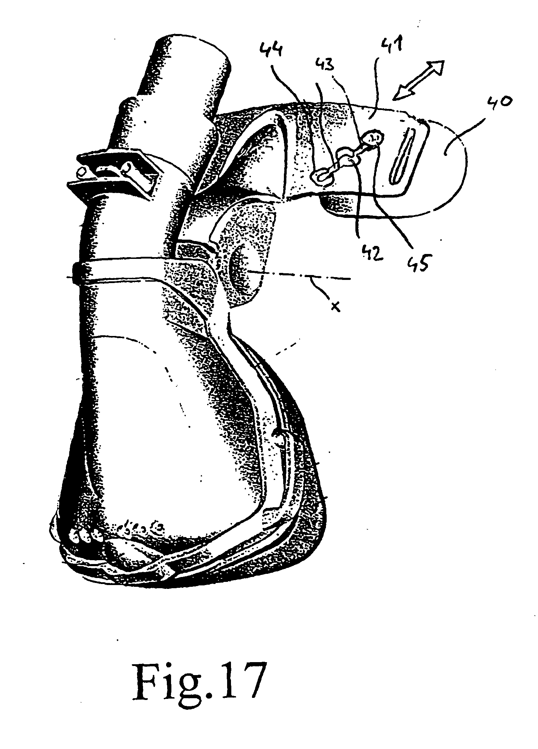

Figur 17 zeigt eine perspektivische Ansicht einer Atemmaske mit schwenkbewegbar angelenkter Stirnauflageeinrichtung und einer Dichtlippeneinrichtung einschließlich zugehörigem Maskenbasisköpers, gemäß Figuren 14 oder 15. Einzelheiten dieser Atemmaske sind in der Patentanmeldung WO03/059427 beschrieben. Diese Atemmaske umfasst Stirnpads 40 die aus einem Elastomermaterial gefertigt sind und an einer Stirnabstützeinrichtung 41 angebracht sind.

Die Befestigung der Stirnpads 40 erfolgt über eine Steckhalterung die eine zentrale Aufnahmeöffnung 42 und anschließende Rasbahnen 43 aufweist, die in Fixiertäler 44 einmünden. In diese Fixiertäler 44 kann ein Rastkopfabschnitt 45 des Stirnpads 40 eintauchen und das Stirnpad 40 in dieser Stellung sichern. Die Rastbahnen 43 sind so ausgelegt, dass das Stirnpad 40 in einer auf den Maskenanwender abgestimmten Position an der Stirnauflageeinrichtung 41 festgelegt werden kann.

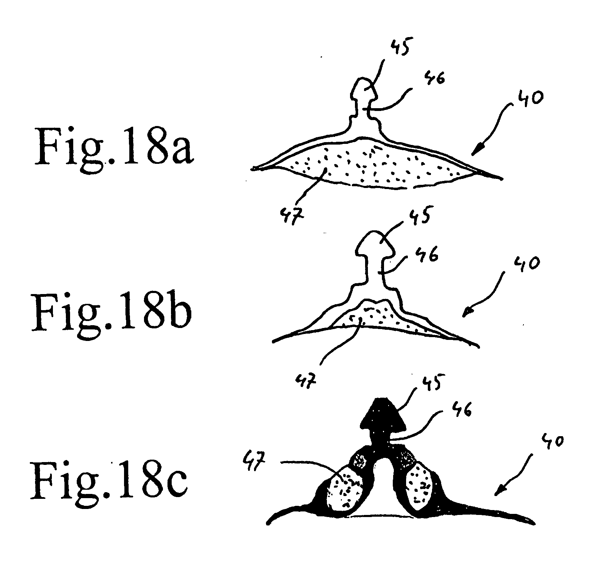

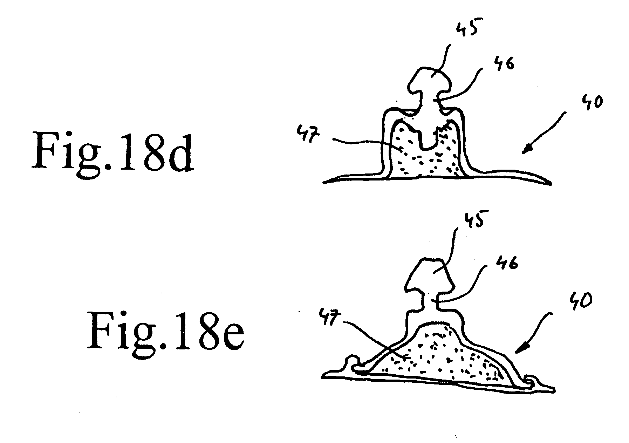

In den Figuren 18a bis 18e sind Schnittansichten von Varianten von Strinauflage-Pads 40 insbesondere zur Verwendung in Verbindung mit einer Atemmaske, beispielsweise gemäß Figur 17 dargestellt, wobei die Stirnauflagepads 40 querschnittsverdickte Zonen aufweisen die mit einem Gel- oder Schaumstoffmaterial gefüllt sind. Die Stirnauflagepads 40 umfassen einen Stützschaft 46 und eine Rastkopfabschnitt 45. Der Stützschaft 46 und der Rastkopfabschnitt 45 sind so ausgebildet, dass diese eine definierte Kippbewegung der zur Auflage auf der Stirnfläche eines Patienten vorgesehenen Auflagezone ermöglichen. Im Bereich dieser Auflagezone sind Profilierungen vorgesehen, die einem Unerdruckaufbau vorbeugen.

Die Pads 40 sind mit einem Gel- oder Schaumstoffmaterialabschnitt 47 versehen. Dieser Abschnitt 47 sowie die diesen umgrenzenden elastomeren Wandungen sind so abgestimmt, dass sich eine definierte Flächenpressungsverteilung und/oder eine definierte Gelenkcharakteristik ergibt.

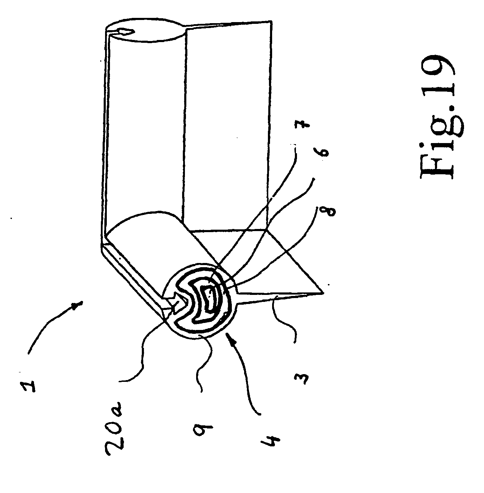

Die in Figur 19 gezeigte Dichtungsstruktur 1 umfaßt eine Dichtlippe 3 und eine sich daran anschließende querschnittsverdickte Zone 4. Im Bereich der querschnittsverdickten Zone 4 ist eine Befestigungsprofilierung 20a vorgesehen, über welche die Dichtungsstruktur an einem Halteprofil befestigbar ist.

Im Bereich der querschnittsverdickten Zone 4 ist das Profilmaterial der Dichtungsstruktur derart verarbeitet, dass die Shore-Härte des Profilmateriales im Kernbereich 6 der querschnittsverdickten Zone niedriger ist als im Randbereich 9 der querschnittsverdickten Zone. Im Kernbereich 6 hat das Profilmaterial im wesentlichen gel-artige Materialeigenschaften. In dem zwischen dem Kernbereich 6 und dem Randbereich 9 liegenden Bereichen 7, 8 ist das Profilmaterial derart vernetzt, dass sich hier zwar höhere Shore-Härten als im Kernbereich 6, jedoch niedrigere Shore-Härten als im Randbereich 9 ergeben. Die Shore-Härte in dem, dem Kernbereich 6 benachbarten Innenbereich 5 ist bei diesem Ausführungsbeispiel niedriger als die Shore-Härte in dem angrenzenden Ringbereich 8.

Die erfindungsgemäße Dichtungsstruktur zeichnet sich dadurch aus, dass diese eine teilausgehärtete, querschnittsverdickte Zone aufweist. Die erfindungsgemäße Dichtungsstruktur ist vorzugsweise aus einem additionsvernetzenden Material, insbesondere Silikonmaterial als Spritzguß- oder Extrusionsprofil gefertigt. Durch Abstimmung des Temperaturprofils einer Formraum oder Extrusionskanalwand können die gewünschten Vernetzungsgrade in einem engen Toleranzbereich festgelegt werden. Gemäß einer besonders bevorzugten Ausführungsform der Erfindung wird die Vernetzungsreaktion im Bereich der querschnittsverdickten Zone aktiv unterbunden, so dass die gewünschten Materialeigenschaften der querschnittsverdickten Zone langzeitstabil aufrechterhalten werden könnten.

Die Vernetzungsreaktion kann insbesondere gestoppt werden, indem die Katalysereaktion durch Zugabe oder Aktivierung eines Katalyse-Blockes unterbunden wird. Die Aktivierung des Katalyse-Blockes kann insbesondere durch UV-Licht durch Mikrowellen, durch elektromagnetische Strahlung oder auch durch Injektion eines Katalyse-Blockes in den Bereich der querschnittsverdickten Zone herbeigeführt werden. Gemäß einer besonders bevorzugten Ausführungsform wir die erfindungsgemäße Dichtstruktür aus einem additionsvernetzenden LSR-Silikon gefertigt, wobei der Bereich der hier dargestellten Dichtlippe durchgehärtet ist, und der sich an die Dichtliope 3 angrenzende querschnittsverdickte Bereich Zonen mit vermindertem Vernetzungsgrad, insbesondere gel-elastischen Eigenschaften aufweist. Die querschnittsverdickte Zone 4 weist in ihrem Randbereich 9 eine vollständig vernetzte Außenhaut auf, wogegen das Material im Kernbereich 6 nahezu unvernetzt ist.

## Patentansprüche

1. Maskenkisseneinrichtung (1) für eine Atemmaske mit einer Aufnahmeöffnung, die in Applikationsposition der Atemmaske wenigstens mit dem Nasen- und/oder Mundöffungsbereich eines Maskenanwenders übereinkommt, und einer aus einem elastomeren Material gebildeten, um die Aufnahmeöffnung umlaufenden Dichtlippe (3), die in Applikationsposition auf der Gesichtsfläche des Maskenanwenders aufsitzt, wobei in dem Maskenkissen wenigstens eine einen Außenbereich aufweisende querschnittsverdickte Zone (4) ausgebildet ist, und der Außenbereich (3b; 9) wesentlich dieselbe Shore-Härte wie die Dichtlippe (3) hat,
**dadurch gekennzeichnet,**
**dass** das Maskenkissenmaterial dieser querschnittsverdickten Zone (4) derart unterschiedliche Materialeigenschaften aufweist, dass die Shore-Härte des Maskenkissens im Randbereich der querschnittsverdickten Zone (4) höher ist als in deren kern- oder zumindest kernnahen Bereich.

2. Maskenkisseneinrichtung nach Anspruch 1, **dadurch gekennzeichnet, dass** die Shore-Härte des Maskenkissens im Bereich der in Applikationsposition im Stirn- oder Nasenrücken-nahen Zonen niedriger als im Bereich der in Applikationsposition den Wangen-, Oberlippen- oder Nasenflügeln-nahen Umfangszonen.

3. Maskenkisseneinrichtung nach Anspruch 2, **dadurch gekennzeichnet, dass** im Kernbereich der querschnittsverdickten Zonen (4) der Werkstoff des Maskenkissens gelartige Materialeigenschaften aufweist.

4. Maskenkisseneinrichtung nach wenigstens einem der Ansprüche 1 bis 3, **dadurch gekennzeichnet, dass** die unterschiedlichen Werkstoffeigenschaftenim Bereich der querschnittsverdickten Zonen (4) durch unterschiedliche Werkstoff-Vernetzungsgrade verursacht sind.

5. Maskenkisseneinrichtung nach wenigstens einem der Ansprüche 1 bis 4, **dadurch gekennzeichnet, dass** die querschnittsverdickten Zonen (4) an die Dichtlippe (3) angrenzen.

6. Maskenkisseneinrichtung nach wenigstens einem der Ansprüche 1 bis 5, **dadurch gekennzeichnet, dass** die querschnittsverdickten Zone (4) in Applikationsposition zumindest abschnittsweise auf der Gesichtsfläche des Maskenanwenders aufsitzt.

7. Maskenkisseneinrichtung nach wenigstens einem der Ansprüche 1 bis 6, **dadurch gekennzeichnet, dass** die querschnittsverdickte Zone (4) in Applikationsposition zumindest abschnittsweise auf der, der Gesichtsfläche des Maskenanwenders abgewandten Innenseite der Gesichts-Dichtlippe (3) aufsitzt.

8. Maskenkisseneinrichtung nach wenigstens einem der Ansprüche 1 bis 7, **dadurch gekennzeichnet, dass** die querschnittsverdickten Zonen aus wenigstens zwei unterschiedlich vorbereiteten Elastomer-Copound-Materialien gebildet sind.

9. Maskenkisseneinrichtung nach wenigstens einem der Ansprüche 1 bis 8, **dadurch gekennzeichnet, dass** diese an einen durch eine Hartschale gebildete Gewölbekörper ansetzbar ausgebildet ist.

10. Maskenkisseneinrichtung nach wenigstens einem der Ansprüche 1 bis 9, **dadurch gekennzeichnet, dass** die Maskenkisseneinrichtung integral mit dem Gewölbekörper ausgebildet ist.

11. Maskenkisseneinrichtung nach wenigstens einem der Ansprüche 1 bis 10, **dadurch gekennzeichnet, dass** die querschnitts-verdickten Zonen (4) zumindest abschnittsweise in Applikationsrichtung federnd nachgiebig aufgehängt ist.

12. Maskenkisseneinrichtung nach wenigstens einem der Ansprüche 1 bis 11, **dadurch gekennzeichnet, dass** die Dichtlippe (3) über eine Faltenstruktur federnd mit der querschnittsverdickte Zone (4) gekoppelt ist.

13. Atemmaske mit einem in Applikationsposition den Nasen- und/oder den Mundbereich eines Maskenanwenders übergreifenden Gewölbekörper und einer Maskenkisseneinrichtung (1) nach wenigstens einem der Ansprüche 1 bis 12.

14. Atemmaske nach Anspruch 13, **dadurch gekennzeichnet, dass** der Gewölbekörper integral mit der Maskenkisseneinrichtung (1) ausgebildet ist.

15. Atemmaske nach Anspruch 13, **dadurch gekennzeichnet, dass** der Gewölbekörper durch eine Hartschale gebildet ist.

16. Verfahren zur Herstellung einer Maskenkisseneinrichtung (1) nach einem der vorhergehenden Ansprüche bei welchen im Rahmen eines Elastomer-Material-Einbringschrittes, das Elastomermaterial in einen Maskenkissenformraum eingebracht wird, wobei die Temperaturverteilung der Formrauminnenwand sowie die Formschliesszeit derart abgestimmt werden, dass das in dem Formraum zu der Maskenkisseneinrichtung (1) vernetzende Elastomermaterial, unterschiedliche Shore-Härten erhält.

17. Verfahren nach Anspruch 16, **dadurch gekennzeichnet, dass** das Temperaturprofil der Formrauminnenwand derart abgestimmt wird, dass die in dem Formraum gebildete Maskenkisseneinrichtung (1) in ihrem Verlauf in Umfangsrichtung unterschiedliche Shore-Härten aufweist.

18. Verfahren nach Anspruch 16 bis 17, **dadurch gekennzeichnet, dass** das Temperaturprofil derart abgestimmt wird, dass die Maskenkisseneinrichtung (1) in einem in Applikationsposition dem Stirn- oder Nasenrücken-nahen Bereich niedrigere Shore-Härten erhält.

19. Verfahren nach Anspruch 16 bis 18, **dadurch gekennzeichnet, dass** das Temperaturprofil der Formrauminnenwand während des Elastomer-Material-Einbringschrittes eine erste Temperatur-Profilcharakteristik aufweist und dass die Temperaturcharakteristik während der Formschliesszeit verändert wird.

20. Verfahren nach Anspruch 19, **dadurch gekennzeichnet, dass** das Temperaturprofil während des Elastomer-Material-Einbringschrittes eine erste insbesondere konstante, Hochtemperaturverteilung hat, und dass während der Formschliesszeit ausgewählte Zonen der Formrauminnenwandung auf niedrigere Temperaturen abgekühlt werden.

## Claims

1. A mask pad device (1) for a breathing mask comprising a receiving opening which in the position of application of the breathing mask coincides at least with the nose and/or mouth opening region of a user of the mask, and a sealing lip (3) which is formed from an elastomer material and which extends around the receiving opening and which in the application position fits on the surface of the face of the user of the mask, wherein at least one zone of thickened cross-section (4) having an outer region is provided in the mask pad, the outer region (3b; 9) having substantially the same Shore hardness than the sealing lip (3) **characterized in that** the mask pad material of said zone of thickened cross-section (4) has different material properties in such a way that the Shore hardness of the mask pad in the edge region of the zone of thickened cross-section (4) is higher than in the region which is at the core or at least near the core of the zone of thickened cross-section.

2. The mask pad device according to claim 1 **characterized in that** the Shore hardness of the mask pad in the region of the zones which in the application position are near the forehead or the bridge of the nose is lower than in the region of the peripheral zones which in the application position are near the cheeks, top lip or sides of the nose.

3. The mask pad device according to claim 2 **characterized in that** in the core region of the zones of thickened cross-section (4) the material of the mask pad has gel-like material properties.

4. The mask pad device according to at least one of claims 1 to 3 **characterized in that** the different material properties in the region of the zones of thickened cross-section (4) are caused by differing degrees of crosslinking of the material.

5. The mask pad device according to at least one of claims 1 to 4 **characterized in that** the zones of thickened cross-section (4) adjoin the sealing lip (3).

6. The mask pad device according to at least one of claims 1 to 5 **characterized in that** the zones of thickened cross-section (4) in the application position are seated at least portion-wise on the surface of the face of the user of the mask.

7. The mask pad device according to at least one of claims 1 to 6 **characterized in that** the zone of thickened cross-section (4) in the application position is seated at least portion-wise on the inward side of the face sealing lip (3), which is remote from the surface of the face of the user of the mask.

8. The mask pad device according to at least one of claims 1 to 7 **characterized in that** the zones of thickened cross-section are formed from at least two elastomer compound materials which are prepared differently.

9. The mask pad device according to at least one of claims 1 to 8 **characterized in that** it is adapted to be fitted to a dish-shaped body formed by a hard shell member.

10. The mask pad device according to at least one of claims 1 to 9 **characterized in that** the mask pad device is formed integrally with the dish-shaped body.

11. The mask pad device according to at least one of claims 1 to 10 **characterized in that** the zones of thickened cross-section (4) are resiliently yieldingly suspended in the application direction at least in a portion-wise manner.

12. The mask pad device according to at least one of claims 1 to 11 **characterized in that** the sealing lip (3) is resiliently coupled by way of a folding structure to the zone of thickened cross-section (4).

13. A breathing mask comprising a dish-shaped body which in the application position engages over the nose and/or mouth region of a user of the mask and a mask pad device (1) according to at least one of claims 1 to 12.

14. The breathing mask according to claim 13 **characterized in that** the dish-shaped body is formed integrally with the mask pad device (1).

15. The breathing mask according to claim 13 **characterized in that** the dish-shaped body is formed by a hard shell member.

16. A process for the production of a mask pad device (1) according to one of the preceding claims in which in the context of a step of introducing elastomer material, the elastomer material is introduced into a mask pad mould cavity, wherein the temperature distribution of the inside wall of the mould cavity and the mould closing time are so controlled that the elastomer material which is crosslinked to form the mask pad device (1) in the mould cavity attains different Shore hardness values.

17. The process according to claim 16 **characterized in that** the temperature profile of the inside wall of the mould cavity is controlled in such a way that the mask pad device (1) formed in the mould cavity has different Shore hardness values in the configuration thereof in the peripheral direction.

18. The process according to claims 16 to 17 **characterized in that** the temperature profile is so controlled that the mask pad device (1) attains lower Shore hardness values in a region which in the application position is near the forehead or the bridge of the nose.

19. The process according to claims 16 to 18 **characterised in that** the temperature profile of the inside wall of the mould cavity during the step of introducing elastomer material has a first temperature profile characteristic and that the temperature characteristic is altered during the mould closing time.

20. The process according to claim 19 **characterised in that** the temperature profile during the step of introducing elastomer material has a first, in particular constant high-temperature distribution, and that during the mould closing time selected zones of the inside wall of the mould cavity are cooled to lower temperatures.

## Revendications

1. Dispositif de coussin (1) pour un masque respiratoire avec une ouverture de réception, qui en position d'application du masque respiratoire vient s'ajuster au moins sur la zone nasale et/ou sur la zone d'ouverture buccale d'un utilisateur du masque, et une lèvre d'étanchéité (3) en matériau élastomère, périphérique à l'ouverture de réception, reposant sur la surface du visage de l'utilisateur du masque en position d'application, où au moins une zone (4) à section transversale épaissie présentant une région extérieure est formée dans le coussin de masque, et où la région extérieure (3b ; 9) a sensiblement la même dureté Shore que la lèvre d'étanchéité (3),
**caractérisé en ce que** le matériau du coussin de masque de ladite zone (4) à section transversale épaissie est doté de propriétés de matériau différenciées, telles que la dureté Shore du coussin de masque dans la région de bord de la zone (4) à section transversale épaissie est supérieure à celle de sa région centrale ou au moins de la région à proximité du centre .

2. Dispositif de coussin pour masque selon la revendication 1, **caractérisé en ce que** la dureté Shore du coussin de masque au niveau des zones proches du front ou de l'arête du nez en position d'application est inférieure à celle des zones périphériques proche des joues, de la lèvre supérieure ou des ailes du nez en position d'application.

3. Dispositif de coussin pour masque selon la revendication 2, **caractérisé en ce que** dans la région centrale de la zone (4) à section transversale épaissie, le matériau du coussin de masque est doté des propriétés d'un matériau de type gel.

4. Dispositif de coussin pour masque selon au moins une des revendications 1 à 3, **caractérisé en ce que** les propriétés de matériau différenciées au niveau des zones (4) à section transversale épaissie sont dues à des degrés différents de réticulation du matériau.

5. Dispositif de coussin pour masque selon au moins une des revendications 1 à 4, **caractérisé en ce que** les zones (4) à section transversale épaissie sont adjacentes à la lèvre d'étanchéité (3).

6. Dispositif de coussin pour masque selon au moins une des revendications 1 à 5, **caractérisé en ce que** la zone (4) à section transversale épaissie repose au moins partiellement sur la surface du visage de l'utilisateur du masque en position d'application.

7. Dispositif de coussin pour masque selon au moins une des revendications 1 à 6, **caractérisé en ce que** la zone (4) à section transversale épaissie repose au moins partiellement sur la face intérieure de la lèvre d'étanchéité (3) de visage éloignée de la surface du visage de l'utilisateur du masque en position d'application.

8. Dispositif de coussin pour masque selon au moins une des revendications 1 à 7, **caractérisé en ce que** les zones à section transversale épaissie sont constituées d'au moins deux matériaux composites élastomères préparés différemment.

9. Dispositif de coussin pour masque selon au moins une des revendications 1 à 8, **caractérisé en ce que** celui-ci est prévu pour être applicable contre un corps bombé formé par une coque dure.

10. Dispositif de coussin pour masque selon au moins une des revendications 1 à 9, **caractérisé en ce que** ledit dispositif de coussin pour masque est réalisé d'une seule pièce avec le corps bombé.

11. Dispositif de coussin pour masque selon au moins une des revendications 1 à 10, **caractérisé en ce que** les zones (4) à section transversale épaissie sont au moins partiellement à suspension élastique dans la direction d'application.

12. Dispositif de coussin pour masque selon au moins une des revendications 1 à 11, **caractérisé en ce que** la lèvre d'étanchéité (3) est élastiquement accouplée par une structure plissée à la zone (4) à section transversale épaissie.

13. Masque respiratoire avec un corps bombé couvrant la zone nasale et/ou la zone buccale d'un utilisateur du masque en position d'application, et avec un dispositif de coussin (1) pour masque selon au moins une des revendications 1 à 12.

14. Masque respiratoire selon la revendication 13, **caractérisé en ce que** le corps bombé est réalisé d'une seule pièce avec le dispositif de coussin (1) pour masque.

15. Masque respiratoire selon la revendication 13, **caractérisé en ce que** le corps bombé est formé par une coque dure.

16. Procédé de fabrication d'un dispositif de coussin (1) pour masque selon l'une des revendications précédentes, où, lors d'une étape d'injection de matériau élastomère, le matériau élastomère est injecté dans une cavité de moule de coussin de masque, la distribution de température de la paroi intérieure de cavité de moule ainsi que le temps de fermeture de moule étant réglés de telle manière que le matériau élastomère réticulant pour former le dispositif de coussin (1) pour masque dans la cavité de moule présente différentes duretés Shore.

17. Procédé selon la revendication 16, **caractérisé en ce que** le profil de température de la paroi intérieure de cavité de moule est réglé de telle manière que le dispositif de coussin (1) pour masque formé dans la cavité de moule présente différentes duretés Shore dans son extension en direction périphérique.

18. Procédé selon les revendications 16 et 17, **caractérisé en ce que** le profil de température est réglé de telle manière que le dispositif de coussin (1) pour masque présente des duretés Shore moindres dans une zone proche du front ou de l'arête du nez en position d'application.

19. Procédé selon les revendications 16 à 18, **caractérisé en ce que** le profil de température de la paroi intérieure de cavité de moule présente une première caractéristique de profil de température lors de l'étape d'injection de matériau élastomère, et **en ce que** la caractéristique de température est modifiée pendant le temps de fermeture du moule.

20. Procédé selon la revendication 19, **caractérisé en ce que** le profil de température présente une première distribution de hautes températures, en particulier constante, lors de l'étape d'injection de matériau élastomère, et **en ce que** des zones sélectionnées de la paroi intérieure de la cavité de moule sont refroidies à des températures moindres pendant le temps de fermeture du moule.
